# EUROPÄISCHE PATENTANMELDUNG

(11) **EP 4 659 738 A1**
(43) Veröffentlichungstag der Anmeldung: **10.12.2025**
(21) Anmeldenummer: 24180190.1
(22) Anmeldetag: 05.06.2024
(51) Int. Cl.: A61K 8/65, A61K 38/17, A61Q 19/00, A61K 8/02, A61P 17/06

(54) **TOPISCHE FORMULIERUNG ENTHALTEND KERATINPARTIKEL ZUR DERMATOLOGISCHEN ANWENDUNG**

(71) Anmelder: Rigi Therapeutics AG, 6045 Meggen (CH)
(72) Erfinder:
(74) Vertreter: Pfenning, Meinig & Partner mbB

(57) **Zusammenfassung**

Die vorliegende Erfindung betrifft eine topische Formulierung enthaltend Keratinpartikel des Proteins beta-Keratin und/oder deren Agglomerate aus Federn tierischen Ursprungs zur dermatologischen Anwendung. Die topische Formulierungen finden Verwendung für die Prophylaxe oder Therapie von Störungen der Barrierefunktion der Haut sowie für die Stabilisierung des Säuremantels der Haut.

## Beschreibung

Die vorliegende Erfindung betrifft eine topische Formulierung enthaltend Keratinpartikel des Proteins beta-Keratin und/oder deren Agglomerate aus Federn tierischen Ursprungs zur dermatologischen Anwendung. Die topische Formulierungen finden Verwendung für die Prophylaxe oder Therapie von Störungen der Barrierefunktion der Haut sowie für die Stabilisierung des Säuremantels der Haut.

Das Stratum corneum (SC) der Epidermis besteht aus mehreren Schichten von keratinisierten Korneozyten, die vom *cornified envelope* ummantelt und durch korneodesmosomale Strukturen verbunden in eine komplexe Lipidmatrix aus flüssigkristallinen, lamellaren Strukturen eingebettet sind. Die Lipidfraktion besteht wesentlich aus Ceramiden (CERs), langkettigen freien Fettsäuren (FFS), Cholesterol (CHOL) und Triglyceriden (TG). Diese bilden in der Interaktion mit Wasser ein komplexes Membrannetzwerk, welches als morphologisches Äquivalent zur Barrierefunktion verstanden wird. CERs bilden dabei, wegen ihrer anisotropen Molekülstruktur und den dadurch begründeten besonderen physikochemischen Eigenschaften, das Rückgrat des Membrannetzwerks, während die anderen lipophilen Bestandteile die Membraneigenschaften, wie Fluidität, Permeabilität und Stabilität modulieren.

Die unterschiedlichen CERs werden aus langkettigen Sphingoidbasen gebildet, die über Amide an Fettsäuren gebunden sind. Es gibt mehrere Klassen von epidermalen CERs, die daraus resultieren, dass es verschiedene Kombinationsmöglichkeiten der vier Typen von Dihydroxy- und Trihydroxy-Sphingoidbasen (Dihydrosphingosin (d18:0), 4-Sphingenin (Sphingosin) (d18:1), 4-Hydroxysphinganin (Phytosphingosin) (t18:0) oder 6-Hydroxysphingosin) mit vier Typen von Fettsäuren (nicht-Hydroxy-, α-Hydroxy-, ω-Hydroxy- oder veresterte ω-Hydroxyfettsäuren) gibt. Darüber hinaus wurde eine weitere Klasse von CERs mit Tetrahydroxy-Sphingoidbasen beschrieben. Außerdem gibt es ω-Hydroxy-CERs, die kovalent an Proteine des *cornified envelope* von Korneozyten gebunden sind. In der wissenschaftlichen Literatur finden sich unterschiedliche Angaben zur quantitativen Verteilung der verschiedenen CER-Klassen innerhalb des SC. Dies lässt auf eine hohe inter- und intraindividuelle Variabilität schließen, deren funktionelle Bedeutung noch nicht vollständig verstanden ist.

Die Bereitstellung der für den Aufbau der interkorneozytären Matrix relevanten Lipide wird durch keratinozytäre Syntheseleistungen gewährleistet. Die synthetisierten Lipide werden in intrazytoplasmatischen Vesikeln, den Lamellarkörperchen (Odland bodies) eingelagert und im Rahmen der Enddifferenzierung des Keratinozyten zum Korneozyten via Exozytose in den interkorneozytären Raum freigesetzt. Darin enthalten sind neben Cholesterol und Phospholipiden vor allem Glykosyl-Ceramide, die nach der Freisetzung durch hydrolytische Enzyme zu FFSs und Ceramiden umgewandelt werden.

Die Barrierefunktion wird zudem wesentlich durch die Quantität der hydrophilen Phase in Mikromilieus bestimmt. Die Verteilung des Wassers innerhalb der Mikrokompartimente des SC lässt sich in mindestens zwei Fraktionen beschreiben. Neben dem sogenannten freien Wasser wird eine Fraktion von gebundenem Wasser definiert. Letztere setzt sich aus einer mobilisierbaren und einer fixierten Subfraktion zusammen. Die Nomenklatur zielt dabei auf den dynamischen Austausch hydrophiler Valenzen zwischen den einzelnen Kompartimenten ab. Unter fixiertem Wasser versteht man vordergründig den Wasseranteil, der in Korneozyten durch starke hygroskopische Kräfte, vermittelt durch proteolytisch generierte Aminosäuren, gebunden ist und quasi für den Austausch nur sehr retardiert zur Verfügung steht. Unter besonderen Bedingungen kann durch schwellfähige Membrananteile gebundenes Wasser liberiert und in die freie Wasserphase überführt werden. Hier wird die funktionell bedeutsame Wasserphase des SC gesehen. Das freie Wasser wird zudem durch hygroskopische Moleküle, die zusammen als "natural moisturizing factor (NMF)" bezeichnet werden, in den einzelnen Mikrokompartimenten gebunden und steht im Austausch mit der Wasserphase der vitalen Epidermis sowie der Umwelt (transepidermaler Wasserfluss). Wesentlicher Bestandteil des NMF sind neben Aminosäuren insbesondere Pyrrolidoncarbonsäuren, Lactat, Harnstoff und anorganische Ionen. Diese werden durch die Keratinozyten synthetisiert und in Abhängigkeit vom Differenzierungsgrad teilweise freigesetzt und teilweise intrakorneozytär wirksam.

Der pH-Wert in den Mikrokompartimenten des SC ist naturgemäß an das Vorhandensein von Wasser sowie an die Zusammensetzung der darin gelösten Bestandteile gebunden. Hierbei ist der Wasserhaushalt innerhalb der Mikrokompartimente des SC und die Verteilung sowie Bioverfügbarkeit der hydrophilen Moleküle von großer Bedeutung. Insbesondere die hygroskopischen Moleküle des natural moisturizing factors (NMF), die unter anderem durch die Proteolyse des Filaggrins intrakorneozytär verfügbar werden, sind in diesem Zusammenhang für die Wasserbindungskapazität des SC von Bedeutung. Innerhalb des SC gibt es einen sigmoidalen pH-Gradienten, dessen mikrotopografisch variierende Höhe für physikalische und metabolische Prozesse von sehr hoher Bedeutung ist. Zu den exogenen Valenzen, die den pH-Wert insbesondere der oberen SC-Anteile bestimmen, tragen neben Umwelteinflüssen Inhaltsstoffe des Schweißes (z.B. Milchsäure) bzw. des Talgs (z.B. FFAs) sowie Metabolite des Mikrobioms der Haut bei. Für die pH-Homöostase des SC scheinen aber insbesondere endogene Einflussfaktoren von essentieller Bedeutung zu sein, zumal diese auch die Pufferkapazität wesentlich bedingen. Der Hautoberflächen-pH wird zwischen 4,5 und 5,0 angegeben und als "Säuremantel der Haut" bezeichnet. Durch sequenzielles *tape stripping* des SC konnte gezeigt werden, dass der pH-Wert von der Oberfläche ausgehend einige korneozytäre Lagen zur Tiefe hin abnimmt, um dann wieder langsam anzusteigen. Dieser, als biphasisch oder der Kurvengeometrie folgend als sigmoidaler pH-Gradient bezeichnete Verlauf, besitzt für metabolische Prozesse innerhalb des SC eine sehr wesentliche Bedeutung.

Der Einfluss von Wasser auf die molekulare Ordnung der Lipidbestandteile des SC lässt sich nicht alleinig durch die physikochemischen Interaktionen der Moleküle und daraus abgeleiteten Membrantheorien erklären. Vielmehr ist der absolute pH-Wert in einzelnen Mikrokompartimenten insbesondere für die Regulation der Aktivitätsoptima verschiedener Enzymsysteme von Bedeutung. Hierbei werden regenerierende Prozesse des Aufbaus der Barrierefunktion, von degenerierenden der Desquamation unterschieden. Für den Aufbau der Membranen ist die Bereitstellung der Lipidbausteine durch Transformation sogenannter *lipid precurser* am Übergang vom Stratum granulosum und im tiefen Schichtdrittel des SC von essentieller Bedeutung. Diese enzymatisch katalysierte Transformation wird für die einzelnen Lipidkomponenten durch unterschiedliche Enzymsysteme mit unterschiedlichen pH-Optima realisiert. Für die Bildung der Ceramide erfolgt die Hydrolyse der β-glycosidischen Bindung von Glucocerebrosiden durch die β-GBA und die Spaltung der Phosphodiesterbindung von Sphingomyelin in Ceramid und Phosphorylcholin durch aSMase. Beide Enzymsysteme besitzen ein Aktivitätsoptimum bei pH 5,5 und einen raschen Aktivitätsabfall bei geringen pH-Abweichungen. Die Bildung von FFAs erfolgt hingegen durch Spaltung einer spezifischen Esterbindung an Phospholipiden durch die sekretorische Phospholipase A2 (sPLA2). Diese besitzt ebenfalls ein sehr schmales Aktivitätsoptimum bei pH 6,8. Cholesterol wird durch die Umwandlung von Cholesterolsulfat, katalysiert durch die Steroidsulfatase (STS), bereitgestellt. Für die STS wird ein breiteres pH-Aktivitätsoptimum (pH 6,0-7.5) angegeben. Für eine effiziente Aktivität der genannten Enzymsysteme der lipid precurser Transformation im SC findet sich somit ein pH-Optimum im sauren Bereich, wobei die Aktivitätstopografie der Enzyme innerhalb der SC-Architektur von großer Bedeutung ist und sich am physiologischen pH-Gradienten orientiert. Im Rahmen der Desquamation des SC kommt es insbesondere durch Serinproteasen (Kallikrein 5 (KLK5) und 7 (KLK7)) sowie die alkalische Ceramidase zur Degradierung von Proteinen (inkl. Enzyme) und Lipiden innerhalb des Systems. Dadurch wird eine regulierte Desquamation ermöglicht, die wiederum die Grundlage für eine Regeneration und Differenzierung des Gesamtsystems bildet. Für diese Enzyme wurde ein pH-Aktivitätsoptimum im leicht basischen (pH 7,0-9,0) beschrieben. Kommt es aufgrund eines pH-Anstiegs im SC zu einer Aktivierung der Serinproteasen, werden ein Abbau von Desmoglein 1 (Corneodesmosomen) und eine verminderte Sekretion der Lamellarkörperchen (Odland bodies) bedingt. Darüber hinaus besitzt der pH-Wert neben einer Vielzahl weiterer Faktoren Einfluss auf die molekulare Organisation innerhalb der Lipidmembranen und somit auf deren Funktionalität. Hierbei ist eine pH-Abhängigkeit der Ordnung lamellarer Strukturen, nicht aber für das *orthorhombic lateral packing* beschrieben. Insgesamt wird aber deutlich, dass der pH-Wert innerhalb des SC unter physiologischen Bedingungen mikrodivers variiert und daraus ein sigmoidaler pH-Gradient resultiert. Diese mikrotopographischen Milieuunterschiede tragen entscheidend zur Regulation der Lipidsynthese und der molekularen Ordnung innerhalb der flüssigkristallinen Strukturen des SC bei. Störungen der Milieubedingungen durch pathologische Verschiebungen des pH-Wertes in einzelnen Mikrokompartimenten des SC bedingen ein Defizit in der Barrierefunktionalität und sind somit von hoher praktischer Relevanz.

Im Rahmen der Desquamation werden im Stratum disjunctum, der obersten Schicht des SC, Korneodesmosomen und Proteine des *cornified envelopes* durch Proteolyse abgebaut. Das prädominante Keratin der Korneozyten ist davon aber direkt nicht betroffen. Deshalb bleibt die Integrität der Korneozyten während der Desquamation sehr lange erhalten. Eine wesentliche Funktion der Korneozyten besteht darin, als residentes Reservoir Wasser über Diffusion des *cornified envelopes* für die Membranbildung im interkorneozytären Raum bereitzustellen. Unter Bedingungen der barriereprotektiven Basistherapie wird die Interaktion von korneozytärem Keratin mit epikutan appliziertem Wasser als unbedeutend angesehen, da eine transzelluläre Passage in relevantem Umfang bisher weder für Wasser, noch für andere hydrophile Substanzen nachgewiesen werden konnte. Als Ursache hierfür wird der *cornified envelope* gesehen, der als Hüll- und Barrieremembran den Korneozyten umgibt.

Für die effektive Substitution der physikochemischen Barriere steht deshalb die Applikation von Wasser im Vordergrund. Von zentraler Bedeutung dabei ist, dass die Hydratisierung des Stratum corneums durch die Applikation eines Topikums den effektiven Verhältnisbereich zum enthaltenen Anteil amphiphiler Lipide (Ceramide) berücksichtigen muss, da sonst eine Überhydratisierung droht, die durch Konfigurationsänderung der Ceramide (*chain flip transition*) eine Reduktion der physikochemischen Barriere zur Folge hat. Diese Gefahr besteht vor allem dann, wenn hygroskopische Substanzen (sog. *humectants)* wie Glycerol oder Harnstoff zum Einsatz kommen und die Wasserbindungskapazität rapide stark erhöht wird. Diese Wasserüberladung (*water overload*) droht insbesondere beim Einsatz von *humectants* in höheren Konzentrationen oder bei deren kombiniertem Einsatz *(humectant paradox).* Daraus ergibt sich, dass trotz deutlich verbesserter Hydratisierung des Stratum corneums bei Anwendung von *humectants,* eine Reduktion des Barriereeffektes resultieren kann. Die Bestimmung der Quantität von Wasser im Stratum corneum ist also für die Barrierefunktionalität alleinig nicht aussagekräftig. Das Ziel muss also eine moderate, bioeffektive Wassersubstitution über möglichst lange Dauer darstellen. Die Messung des Gesamtgehaltes des Wassers im Stratum corneum durch Impedanzmessung im Rahmen der Corneometrie ist dafür letztlich also unzureichend. Es gilt also einen mittleren "produktiven" Wassereintrag zu realisieren.

Weiterhin muss berücksichtigt werden, dass die im Rahmen der Metamorphose der applizierten Matrix anteilig liberierte Wasserphase ohne Einsatz von *humectants* in erheblichem Umfang evaporiert und nicht Barriere-protektiv wirksam wird. Dieser Anteil wird bei Einsatz von *humectants* erheblich reduziert, allerdings diffundieren die hygroskopischen Moleküle, entsprechend des sich durch die Verdunstung einstellenden Wassergefälles, in tiefere Schichten und können bei Überdosierung auch einen *steal effect* auslösen. Somit kann Wasser, entgegen des Verwendungszweckes des Topikums, auch aus dem Zielkompartiment gebunden und somit der Bioverfügbarkeit mit Bezug auf die Membranbildung entzogen werden. Unter physiologischen Bedingungen bilden Korneozyten, als örtlich stabile (residente) Mikrokompartimente, eine Art Wasserreservoir und sichern die dosierte, aber stetige Verfügbarkeit von Wasser zur Membranbildung.

Bisherige Substitutionsstrategien basieren auf dem Einsatz von hydrophilen Phasen, insbesondere auf saurem Wasser, sowie *humectants* und polaren Lipiden, um die Membranbildung zu fördern und die Bedingungen für die Regeneration des SC zu verbessern. In Anlehnung an die physiologische Einbindung des keratinhaltigen Korneozyten als intrinsische Wasserspeicher- und Verteilungskomponente sowie Matrix-stabilisierende Struktureinheit wäre dessen funktionelle und morphologische Substitution zielführend. Vor diesem Hintergrund wurden Keratinpartikel aus Vogelfedern extrahiert. Dabei handelt es nicht um hydrolysiertes Keratin, sondern um intakte Keratinmoleküle, die als langgestreckte Filamente die Besonderheit der Häufung von hydrophilen und lipophilen Aminosäuresequenzen aufweisen. Aufgrund ihrer molekularen Masse können sie das SC nicht permeieren und sind nach der Applikation ortsständig (resident) und binden sowohl Wasser als auch *humectants* in Analogie zum natürlichen Feuchthaltefaktor (NMF).

Der Einsatz von Keratin für die medizinische bzw. kosmetische Indikation ist aus folgenden Druckschriften bekannt.

Bislang verfügbares Keratin wird aufgrund von mikrobieller, säure-oder basenbedingter Hydrolyse in kleinere Peptidstrukturen abgebaut und extrahiert und liegt nicht mehr als intaktes und vollständiges Protein Keratin vor (Shavandi, A. et al., Biomater. Sci. 2017, 5, 1699-1735; Gupta, A. et al., J. Chem. Chem. Eng. 2012, 6, 732-737).

Diese Keratin-Peptide sind in ihren Sequenzen kurzkettig und weisen andere Eigenschaften auf (z.B. Molekülgröße, Quellverhalten) als das native, hoch-sequente Keratin-Protein, welches hier erstmalig gewonnen werden konnte.

Ausgehend hiervon war es Aufgabe der vorliegenden Erfindung, topische Formulierungen bereitzustellen, die eine dynamische Stabilisierung von Biomembranen, insbesondere Ceramid-basierte Stratum corneum-Lipidmodellmembranen ermöglichen.

Diese Aufgabe wird mit der topischen Formulierung mit den Merkmalen des Anspruchs 1 sowie deren Verwendung gemäß Anspruch 13 gelöst. Die weiteren abhängigen Ansprüche nennen bevorzugte Ausführungsformen.

Erfindungsgemäß wird eine topische Formulierung, die Keratinpartikel des Proteins beta-Keratin und/oder deren Agglomerate aus Federn tierischen Ursprungs enthält, zur dermatologischen Anwendung bereitgestellt.

Die vorliegende Erfindung betrifft somit den Einsatz von Keratinpartikeln des Proteins beta-Keratin, die durch chemische Denaturierung aus Vogelfedern als Protein Keratin extrahiert und in flüssigen, halbfesten oder festen Zubereitungen zur epikutanen Applikation eingesetzt werden.

Die Federn tierischen Ursprungs sind vorzugsweise ausgewählt aus der Gruppe bestehend aus Vogelfedern, insbesondere Federn von Hühnern, Gänsen, Puten, Enten, Truthähnen, Fasanen, Straußen, Nandus, Emus, Wachteln und Mischungen hiervon.

Als Keratinquelle wurden verschiedene tierische Materialien ausgewählt, die aber aufgrund ihres Proteinvernetzungsgrades unterschiedlich geeignet erscheinen. Nach Voruntersuchungen mit unterschiedlichen Keratin-enthaltenden Biomaterialien zeigte sich eine besondere Eignung von Vogelfedern. Neben praktischen Aspekten der Verfügbarkeit und einfacher Verarbeitung wurden vor allem biochemische Aspekte erarbeitet, die eine präferenzielle Verwendung von Federn begründen. Das in Federn enthaltene Keratin entspricht vorwiegend beta-Keratin, welches als Polypeptidkette mit einer beta-Faltblattstruktur ausgestattet ist, aus Filamenten von 3-4 nm besteht und eine molekulare Masse von ca. 10-22 kDa aufweist. Im Unterschied zu alpha-Keratin bei Säugetieren finden sich bei beta-Keratin wenige, aber funktionell bedeutende Unterschiede in der Primärsequenz. So bildet beta-Keratin weniger Makrofibrillen als alpha-Keratin und zeigt ein regelmäßigeres Ordnungs- und Packungsverhalten. Diese Unterschiede bieten somit günstigere Voraussetzungen für eine Standardisierung der Keratinisolierung und der daraus resultierenden Produkteigenschaften. Dabei gilt es insbesondere die häufig praktizierte Hydrolysierung von Keratin zu vermeiden, um intakte Keratinproteine zu isolieren und die Bildung von Keratinbruchstücken, Aminosäuren und Peptide gezielt zu vermeiden.

Es ist bevorzugt, dass die Keratinpartikel ein mittleres Molekulargewischt im Bereich von 5 bis 50 kDa, bevorzugt von 10 bis 30 kDa, gemessen mittels Natriumdodecylsulfat-Polyacrylamidgelelektrophorese (SDS-PAGE), aufweist.

Es ist bevorzugt, dass das Protein beta-Keratin in seiner Sekundärstruktur als beta-Faltblatt vorliegt. Im Unterschied zum alpha- (= soft fiber) Keratin zeigt beta- (= hard fiber) Keratin einen hohen Anteil an eng zusammengedrehter und durch Disulfidbrücken stabilisierter beta-Faltblattstrukturen, die eine hohe Stabilität des hard fiber-Keratins gewährleisten.

Es ist bevorzugt, dass die Keratinpartikel und/oder deren Agglomerate in der topischen Formulierung eine Partikelgröße im Bereich von 10 bis 120 µm, bevorzugt von 25 bis 100 µm, gemessen mittels dynamischer Lichtstreuung (nach DIN ISO 22412:2018-09) (Gerät Zetasizer ZEN3600, Malvern Panalytical Instruments), aufweisen.

Eine weitere bevorzugte Ausführungsform sieht vor, dass die topische Formulierung Additiva enthält. Diese sind bevorzugt ausgewählt aus der Gruppe bestehend aus
- Kohlenhydraten, insbesondere Saccharose, Lactose, Glucose, Fructose, Mannitol, Sorbitol und Süßstoffen wie Saccharin-Natrium, Natriumcyclamat, Aspartam, Stärke und modifizierte Stärke, Cyclodextrine und/oder Mischungen hiervon,
- Ionische Tenside, wie anionische bzw. polyanionische Tenside, insbesondere Natriumdodecylsulfat, Natriumcetylstearylsulfat, Cetylstearylalkohol (emulgierend), Natriumdioctylsulfosuccinat und/oder kationische Tenside, insbesondere Metallseifen und/oder Mischungen hiervon,
- Nichtionische Tenside, insbesondere Fettalkohole und Sterole, Sortitanfettsäureester, Polyoxyethylen-Sorbitanfettsäureester, Polyoxyethylen-Fettsäureglyceride, Macrogol-1000-glycerolmo-nofettsäureester, Polyoxyethylen-Fettsäureester, Polyoxyethylen-Fettalkoholether, Glycerolfettsäureester, Saccharosefettsäureester, Poloxamere und/oder Mischungen hiervon,
- Gelbildner, insbesondere Polyacrylate, Poloxamer, Cellulosederivate, wie Methycellulose, Methylhydroxyproylcellulose Hydroxypropylcellulose, Hydroxyethylcelluloseund/oder Ethylcellulose, Carmellose-Natrium und/oder Mischungen hiervon,
- Verdickungsmittel, insbesondere Tragant, Xantham, arabisches Gummi, Guargalactomannan, Alginate, Bentonit, und/oder Mischungen hiervon,
- Filmbildnern, insbesondere Methacrylsäure-Acrylaten, Polyvidon, Polyvinylalkohol und/oder Mischungen hiervon,
- Polymeren insbesondere Macrogole, Gelatine und/oder Mischungen hiervon,
- Konservierungsstoffe, insbesondere Methyl-4-hydroxybenzoat, Propyl-4-hydroxybenzoat, Sorbinsäure, Kaliumsorbat, Propylenglykol und/oder Mischungen hiervon,
- Antioxidativa, insbesondere Tocopherolacetat, Carotinoide, Flavonoide, Ascorbinsäure und/oder Mischungen hiervon,
- Duftstoffe
- Puffersubstanzen, insbesondere Natriumlactat, Glycolsäure und/oder Mischungen hiervon.

Es ist weiter bevorzugt, dass die topische Formulierung kosmetische Wirk- und Hilfsstoffe enthält, bevorzugt ausgewählt aus der Gruppe bestehend aus Aminosäuren, Peptide, Proteine, Harnstoff, Glycerol, Hyaluronsäure, Vitamine, Zuckern- und zuckerähnlichen Derivaten, Extrakten oder Wachsen aus Pflanzen oder von Tieren stammenden Teilen und Mischungen hiervon.

Die dermatologische Anwendung betrifft vorzugsweise die Stabilisierung einer Biomembran, insbesondere der Membranstruktur des Stratum corneum durch den Einbau des beta-Keratins, wodurch vorzugsweise eine Prophylaxe oder Therapie von Störungen der Barrierefunktion der Haut ermöglicht wird.

Die dermatologische Anwendung betrifft weiterhin bevorzugt eine mechanische Stabilisierung von Biomembranen, insbesondere von Ceramidmembranen des Stratum corneums, durch Erhöhung des Kollapswiderstandes der Membran durch den Einbau des beta-Keratins.

Ebenso betrifft die dermatologische Anwendung vorzugsweise eine Stabilisierung der äußeren Schicht des Tränenfilms durch den Einbau des beta-Keratins.

Es ist bevorzugt, dass die dermatologische Anwendung an Lebewesen, insbesondere an Menschen und/oder Tieren erfolgt, insbesondere für die Haut, Schleimhaut oder Hautanhangsgebilde, bevorzugt Nägeln und Haaren.

Die erfindungsgemäßen topischen Formulierungen liegen bevorzugt als
- gasförmige Grundlagen, insbesondere Aerosole und/oder Gasaphronen
- flüssige Grundlagen, insbesondere Lösungen, Emulsionen, Suspensionen, Schäume und/oder Kolloiden,
- halbfeste Grundlagen, insbesondere Salben, Cremes, Gele, Pasten, Kolloiden und/oder Suppositorien oder
- feste Grundlagen, insbesondere Pulver, Puder, Tabletten, Granulate, Pellets, Kapseln, Pflaster, Wundauflagen und Verbandsmittel, Textilfasern und/oder Inserts
vor.

Die vorliegende Erfindung betrifft somit den Einsatz von Keratinpartikeln des Proteins beta-Keratin, die durch chemische Denaturierung aus Vogelfedern als Protein Keratin extrahiert und in flüssigen oder halbfesten Zubereitungen zur epikutanen Applikation eingesetzt werden.

Erfindungsgemäß werden die Keratinpartikel aus Federn tierischen Ursprungs hergestellt nach einem Verfahren, bei dem
a) eine Extraktion des Proteins beta-Keratin, insbesondere beta-Keratin in seiner Sekundärstruktur als beta-Faltblatt, in einer eine chemische Denaturierung auslösenden Extraktionslösung enthaltend mindestens ein Denaturierungsagens, mindestens eine Base, mindestens ein Reduktionsmittel und mindestens eine Puffersubstanz erfolgt,
b) die Extraktionslösung aus Schritt a) einer Filtration unterzogen wird, bei der eine kolloidale Lösung der Keratinpartikel des Proteins beta-Keratin erhalten wird,
c) die kolloidale Lösung aus Schritt b) per Gefriertrocknung, Sprühtrocknung, Vakuumtrocknung, Lufttrocknung, Wärmetrocknung, Infrarottrocknung und/oder Mikrowellentrocknung getrocknet wird unter Erhalt eines Keratinpartikel enthaltenden Pulvers.

Im Unterschied zu bisherigen Verfahren wurde auf eine Hydrolyse zur Extraktion verzichtet und stattdessen das Protein über das Brechen der Disulfidbrücken für die Extraktion zugänglich gemacht. Dadurch bleiben die Primärstruktur und die Sekundärstruktur des Proteins Keratin als beta-Faltblatt erhalten.

Bei der Gewinnung von Keratin aus Wollhaaren werden alpha-und beta-Keratin gewonnen, sogenanntes "soft fiber" (alpha) oder "hard fiber"(beta)-Keratin, also zwei unterschiedliche Arten des Keratins. Die hier durchgeführte Extraktion mit Harnstoff (Urea), führt im alkalischen Bereich dazu, dass die native Form des beta-Keratins als gesamtes Protein aus den Federn gewonnen wird und so den hohen Anteil an seiner Sekundärstruktur, dem beta-Faltblatt, behält, der die hohe Wasserbindekapazität dieses Keratins bedingt. Beta-Keratin wird insbesondere aus Vogelfedern gewonnen. Es ist reich an den Aminosäuren Glycin und Alanin und besitzt wenig Cystein, Prolin und Hydroxyprolin.

Dabei wird die Interaktion des Keratins mit hydrophilen Substanzen als Reservoir zur gezielten Beeinflussung deren Pharmakokinetik genutzt. Durch die Kombination des Keratins mit Wasser, Aminosäuren, hygroskopischen Substanzen, Peptiden und/oder Proteinen soll zudem eine Substitution der physikalischen Barriere erzielt werden.

Vorzugsweise weist die Extraktionslösung einen pH-Wert von 8 bis 13, bevorzugt von 9 bis 12 und besonders bevorzugt von 10 bis 11 auf.

Es ist bevorzugt, dass die Keratinpartikel und/oder deren Agglomerate in der kolloidalen Lösung eine Partikelgröße im Bereich von 10 bis 120 nm, bevorzugt im Bereich von 25 bis 100 nm, gemessen mittels dynamischer Lichtstreuung (nach DIN ISO 22412:2018-09) (Gerät Zetasizer ZEN3600, Malvern Panalytical Instruments), aufweisen.

Die Keratinpartikel in Pulverform weisen eine Partikelgröße im Bereich von 1 bis 50 µm, bevorzugt von 10 bis 30 µm, gemessen mittels dynamischer Lichtstreuung (nach DIN ISO 22412:2018-09) (Gerät Zetasizer ZEN3600, Malvern Panalytical Instruments), auf. Die Agglomerate der Keratinpartikel in Pulverform weisen eine Agglomeratgröße im Bereich von 10 bis 500 µm, bevorzugt von 30 bis 250 µm, gemessen mittels dynamischer Lichtstreuung (nach DIN ISO 22412:2018-09) (Gerät Zetasizer ZEN3600, Malvern Panalytical Instruments), auf.

Es ist bevorzugt, dass das mindestens eine Denaturierungsagens ausgewählt ist aus der Gruppe bestehend aus Harnstoff, Thioharnstoff, Guanidinhydrochlorid, Natriumdodecylsulfat (SDS) und Mischungen hiervon.

Es ist weiter bevorzugt, dass die mindestens eine Base ausgewählt ist aus der Gruppe bestehend aus Natriumhydroxid, Kaliumhydroxid und Mischungen hiervon.

Das mindestens ein Reduktionsmittel ist vorzugsweise ausgewählt aus der Gruppe bestehend aus β-Mercaptoethanol, Cysteaminen, Cysteine, Glutathion, Natriumdisulfit, Natriumsulfid, Natriumhydrogensulfit, Natriumdithionit, Natriumthiosulfat, Dithiotreitol (DTT), Thioglykolsäure und ihre Salze, Thioharnstoff, Tricarboxyethylphosphan (TCEP) und andere Phosphane, Ammoniumchlorid und Mischungen hiervon.

Es ist bevorzugt, dass die mindestens eine Puffersubstanz ausgewählt ist aus der Gruppe bestehend aus Tris(hydroxymethyl)aminomethan, Natriumdodecylsulfat (SDS), Tris/Salzsäure (HCl), Ethylendiamintetraessigsäure (EDTA)/Tris, Kaliumchlorid-Natriumhydroxid (KCI-NaOH), Natriumhydrogencarbonat (NaHCO₃), Dithiotreitol (DTT)/Tris und Mischungen hiervon.

Es ist bevorzugt, dass die Extraktionslösung mindestens eine der folgenden chemischen Komponenten enthält:
- mindestens ein Oxidationsmittel ausgewählt aus der Gruppe bestehend aus Wasserstoffperoxid, Kaliumpermanganat, Natriumperborat, Peroxyessigsäure, Perameisensäure und Mischungen hiervon,
- mindestens eine Säure ausgewählt aus der Gruppe bestehend aus Salpetersäure, salpetrige Säure, hypo-und hyperhalogenige Säuren und Mischungen hiervon,
- mindestens ein ionisches Agens ausgewählt aus der Gruppe bestehend aus 1-Butyl-3-methyl-imidazolium (BMIM)-chlorid, 1-Butyl-3-methyl-imidazolium (BMIM)-Bromid, 1-Butyl-3-methyl-imida-zolium (BMIM)-tetrafluoroborat, Amidchlorid und Mischungen hiervon,

Eine bevorzugte Ausführungsform des erfindungsgemäßen Verfahrens sieht vor, dass bei der Extraktion in Schritt a) mindestens einer der folgenden Schritte erfolgt:
- eine mechanische Zerkleinerung, insbesondere durch Zermahlen via Ultraschall, bevorzugt im Frequenzbereich von 20 bis 50 Hertz, mit einer Kugelmühle und/oder mit einer Schneidmühle, bevorzugt Ultra-Turrax, wobei die mechanische Zerkleinerung nach Siebanalyse bis auf eine Partikelgröße (d50) von 0,1 bis 5,0 mm, bevorzugt 0,2 bis 1,0 mm, erfolgt,
- eine thermische Denaturierung, insbesondere bei Temperaturen von 70°C bis 150°C, und/oder elektrochemische Denaturierung,
- eine Fällung der Extraktionslösung aus Schritt a), insbesondere ausgelöst durch eine pH-Änderung, Zugabe eines Co-Solvens und/oder eines Salzes,
- eine mikrobielle und enzymatische Extraktion über
   - gram negative Bakterien ausgewählt aus der Gruppe bestehend aus *Stenotrophomonas* sp., *Chrysebacterium* sp., *Vibrio* sp. und Mischungen hiervon,
   - gram positive Bakterien ausgewählt aus der Gruppe bestehend aus *Bacillus sp., Kocuria rosea* und Mischungen hiervon
   - saprophytische und/oder parasitische Pilze und/oder
   - Mischungen hiervon,
- Behandlung mit Mikrowellenstrahlung, insbesondere Mikrowellenstrahlung bis zu 960 Watt und 2450 Hertz,
- Einsatz von Stromexplosion und/oder überkritischem Wasser und/oder.
- Kombinationen hiervon.

Es ist bevorzugt, dass das Protein beta-Keratin in seiner Sekundärstruktur als beta-Faltblatt vorliegt. Im Unterschied zum alpha- (= soft fiber) Keratin zeigt beta- (= hard fiber) Keratin einen hohen Anteil an eng zusammengedrehter und durch Disulfidbrücken stabilisierter beta-Faltblattstrukturen, die eine hohe Stabilität des hard fiber-Keratins gewährleisten.

Vorzugsweise erfolgt die Filtration über Dialyse und/oder Ultrafiltration (Cross-Flow-Filtration).

Es ist bevorzugt, dass zur Stabilisierung der kolloidalen Lösung mit/ohne Wirk- und/oder Hilfsstoffe diese als liposomale Systeme in uni- oder multilamellare Vesikel unterschiedlicher oder einheitlicher Größe eingebaut sind durch Zusatz von amphiphilen Molekülen. Diese amphiphilen Moleküle sind bevorzugt ausgewählt aus der Gruppe bestehend aus:
- Phospholipiden, bevorzugt Lecithin, DODAB, DPPC, DSPC, DSTAP und/oder Mischungen hiervon,
- kationischen Lipiden, bevorzugt ALC-0315,
- PEGylierte Lipide, bevorzugt ALC-0159
- Prostaglandine und -modifikationen, bevorzugt PGE1, PGD2, PGE2, 15-keto PGE1 und/oder Mischungen hiervon,
- Ceramiden, bevorzugt ausgewählt aus der Gruppe bestehend aus Ceramiden der Kopfgruppen NS, NH, NP, NDS, AS, AH, ADS, AP der Kettenlängen C₁₀ bis C₂₆ und/oder Mischungen hiervon,
- Ceramiden, bevorzugt ausgewählt aus der Gruppe bestehend aus Ceramiden der Kopfgruppen EOS, EOH, EOP der Kettenlängen C₁₀ bis C₃₂ und/oder Mischungen hiervon,
- Cholesterol, Cholesterolderivaten und/oder Mischungen hiervon,
- Fettsäuren, bevorzugt ausgewählt aus der Gruppe bestehend aus Fettsäuren der Kettenlängen C₁₀ bis C₃₂ und/oder Mischungen hiervon.

Anhand der nachfolgenden Figuren und Beispiele soll der erfindungsgemäße Gegenstand näher erläutert werden, ohne diesen auf die hier gezeigten spezifischen Ausführungsformen einschränken zu wollen.
- Fig.1: zeigt anhand einer schematischen Darstellung den Aufbau eines Langmuir-Trogs zur Bestimmung der Oberflächenaktivität
- Fig.2: zeigt anhand eines Diagramms die Isothermen von verschiedenen Ceramiden
- Fig. 3: zeigt anhand eines Diagramms die Isothermen von verschiedenen Mischungen von Ceramiden mit Cholesterol (CHOL) und Stearinsäure (SA) als freier Fettsäure
- Fig. 4: zeigt Spektren der Infrarot-Refelxions-Absorptions-Spektroskopie (IRRAS) von verschiedenen Ceramiden
- Fig. 5: zeigt Spektren der Infrarot-Refelxions-Absorptions-Spektroskopie (IRRAS)von verschiedenen Mischungen von Ceramiden mit Cholesterol (CHOL) und Stearinsäure (SA) als freier Fettsäure
- Fig. 6: zeigt anhand eines Diagramms die Änderung der Oberflächenspannung in Abhängigkeit von der Konzentration einer erfindungsgemäßen Keratinlösung
- Fig. 7: zeigt anhand eines Diagramms die Druck-Flächen-Isotherme von verschiedenen Ceramiden
- Fig. 8: zeigt anhand eines Diagramms die Druck-Flächen-Isotherme von verschiedenen Mischungen von Ceramiden mit Cholesterol (CHOL) und Stearinsäure (SA) als freier Fettsäure
- Fig. 9: zeigt anhand eines Diagramms die Änderung des Oberflächendrucks einer keratinhaltigen Subphase über die Zeit nach Spreitung eins SCLipidfilms einer Mischung von Ceramiden mit Cholesterol (CHOL) und Stearinsäure (SA) als freier Fettsäure
- Fig. 10: zeigt anhand einer schematischen Darstellung die lamellaren (obere Reihe) und lateralen (untere Reihe) Anordnungen von Ceramiden für unterschiedliche Lipidpackungen (orthorhomisch, hexagonal und flüssigkristallin)
- Fig. 11: zeigt Röntgendiffraktionsspektren unter streifendem Einfall (GIXD) (oben) und ein Intensitätsspektrum (unten) für Monoschichten aus dem Ceramid α-Hydroxy-Phytosphingosin [AP] mit Cholesterol (CHOL) und Stearinsäure (SA) auf Wasser (A1) und Keratin (A2)
- Fig. 12: zeigt Röntgendiffraktionsspektren unter streifendem Einfall (GIXD) (oben) und ein Intensitätsspektrum (unten) für Monoschichten aus dem Ceramid nicht-6-Hydroxysphingosin [NH] mit Cholesterol (CHOL) und Stearinsäure (SA) auf Wasser (H1) und Keratin (H2)
- Fig. 13a und b: zeigen anhand zweier Diagramme die Wasseraufnahme einer erfindungsgemäßen topischen Formulierung im Vergleich zu einer Formulierung enthaltend hydrolysiertes Keratin
- Fig. 14a und b: zeigen anhand zweier Diagramme die Wasserabgabe einer erfindungsgemäßen topischen Formulierung im Vergleich zu einer Formulierung enthaltend hydrolysiertes Keratin

### Beispiel 1

Als Keratinquelle wurden verschiedene tierische Materialien ausgewählt, die aber aufgrund ihres Proteinvernetzungsgrades unterschiedlich geeignet erscheinen. Nach Voruntersuchungen mit unterschiedlichen Keratin-enthaltenden Biomaterialien zeigte sich eine besondere Eignung von Vogelfedern. Neben praktischen Aspekten der Verfügbarkeit und einfacher Verarbeitung wurden vor allem biochemische Aspekte erarbeitet, die eine präferenzielle Verwendung von Federn begründen. Das in Federn enthaltene Keratin entspricht vorwiegend beta-Keratin, welches als Polypeptidkette mit einer beta-Faltblattstruktur ausgestattet ist, aus Filamenten von 3-4 nm besteht und eine molekulare Masse von ca. 10-22 kDa aufweist. Im Unterschied zu α-Keratin bei Säugetieren finden sich bei beta-Keratin wenige, aber funktionell bedeutende Unterschiede in der Primärsequenz. So bildet beta-Keratin weniger Makrofibrillen als α-Keratin und zeigt ein regelmäßigeres Ordnungs- und Packungsverhalten. Diese Unterschiede bieten somit günstigere Voraussetzungen für eine Standardisierung der Keratinisolierung und der daraus resultierenden Produkteigenschaften. Dabei gilt es insbesondere die häufig praktizierte Hydrolysierung von Keratin zu vermeiden, um intakte Keratinproteine zu isolieren und die Bildung von Keratinbruchstücken, Aminosäuren und Peptide gezielt zu vermeiden. Für eine effektive und standardisierte Keratinisolierung wurde, basierend auf den biochemischen Besonderheiten von beta-Keratin, ein gesondertes Verfahren entwickelt. Die verwendeten Hühnerfedern wurden zunächst mit Wasser und Seife gereinigt, mit 70%-igem Ethanol desinfiziert und anschließend bei Raumtemperatur getrocknet. Nach der Zerkleinerung der gesäuberten und getrockneten Federn in einer Schneidmühle (Typ Retsch SM 100 comfort) erfolgte eine Homogenisierung des gesamten Materials. Mittels einer Soxhlet-Apparatur wurde das Federhomogenat entfettet. Für die Extraktion des Keratins wurde das entfettete Federmaterial zu einem Extraktionspuffers gegeben und 48 h lang extrahiert. Anschließend wurde der Extrakt zentrifugiert und das Sediment verworfen. Der erhaltene reine Extrakt wurde mit Wasser verdünnt und einer Filtration mit einem Cut off von 10.000 NMWC unterzogen. Mittels Sprühtrocknung wurde das Dialysat zu einem Pulver mit einer Partikelgröße von < 25 µm verarbeitet.

Zur Bestimmung der kolloidalen Größe der Keratinpartikel (1 mg/ml deionisiertes Wasser) wurden Messungen per dynamischer Lichtstreuung (DLS) mittels dem Gerät Zetasizer ZEN3600 von Malvern Panalytical Instruments nach DIN ISO 22412:2018-09 durchgeführt. Die Analysemethode ermöglicht die Charakterisierung von Partikelgrößen in Suspensionen und Emulsionen, bei der das an den Partikeln gestreute Licht eines Lasers ermittelt wird. Ziel war die Erfassung der Partikelgröße des Proteins im kolloidalen Zustand. Für die Messung der DLS wurde 1 ml einer 0,1 mM kolloidalen Keratin-Lösung in eine Einmalküvette aus Polystyrol pipettiert und anschließend in das auf 25 °C temperierte Küvetten-Modul des Zetasizer Lab überführt. Die Partikelgröße wurde mit Hilfe des automatischen Analysemodus (General purpose) und rückwärtigen Lichtstreuung in einem Winkel von 173° ermittelt. Jede Messung erfolgte als Triplikat und umfasste jeweils 15 Messzyklen pro Durchlauf. Die erfassten Messdaten pro Probe wurden anschließend gemittelt. Insgesamt wurden drei Proben mittels DLS untersucht. Im Rahmen der DLS wurde der Polydispersitätsindex (Pdl), welcher angibt wie homogen die Partikel innerhalb der Probe verteilt sind, die Größe der Partikel (in nm) und der prozentuale Anteil derjeweiligen Partikelgröße in Relation zum gesamten Probeninhalt, bestimmt.

Zur Bestimmung der Molekulargröße (Molekulargewicht) der extrahierten Keratinpartikel wurde eine Natriumdodecylsulfat-Polyacrylamidgelelektrophorese (SDS-PAGE) in einer SDS-Gelkammer (Mini Gel Tank von Invitrogen) durchgeführt. Die Proben wurden nach dem Herstellerprotokoll NuPAGE^{®} Bis-Tris Mini Gel Electrophoresis (Thermo Fisher) denaturiert. Hierfür wurden 2 µl Probe, 2,5 µl NuPAGE^{®} LSD Sample Buffer (4x), 1 µl NuPAGE^{®} LSD Reducing Agent (10x) und 6,5 µl DI-Wasser zusammengegeben und für 10 min bei 95 °C inkubiert. Zur Durchführung der Gelelektrophorese wurde die Gelkammer mit einem 1 x Tris-Tricin-Laufpuffer (1,2 M Tris, 0,8 M Tricin, 2 % SDS) gefüllt. Anschließend wurde das Tris-Tricin-Gel (Novex 10-20 % Tricin Gele von Invitrogen (Thermo Fisher Scien-tific), LOT 20101945, REF EC6625BOX) eingesetzt und der Kamm aus dem Gel entfernt. Je 10 µl der vorbereiteten Proben und jeweils 2 µl des Markers wurden in die entsprechenden Geltaschen gegeben. Die Gelelektrophorese wurde bei 130 V und 250 mA für 1,5 Stunden durchgeführt. Das Gel wurde anschließend aus der Kammer entfernt und der Laufpuffer verworfen. Die Plastik-Gel-Halterung wurde aufgebrochen und das Tris-Tricin-Gel in einen Behälter mit Coomassie-Brilliant-Blue G250 Färbemittel (2 ml 5 % Coomassie-Lösung, 3 ml Ortho-Phosphatsäure, 20 ml Ethanol, 10 g Ammoniumsulfat, 65 ml dH2O) überführt. Das Gel wurde in der Lösung über Nacht auf dem Schüttler inkubiert. Im Anschluss wurde die Färbelösung entfernt. Zur Entfärbung wurde das Gel mehrmals mit destilliertem Wasser, unter leichtem Schütteln, gespült.

Es wurde versucht, die Funktionalität eines Korneozyten nachzustellen. Dazu wurde aus verschiedenen natürlichen Keratinquellen Keratin extrahiert und entsprechende Partikel generiert, die zunächst als mechanische Stabilisierung von Lipidmatrices in verschiedenen halbfesten Zubereitungen untersucht wurden. Dabei war beabsichtigt, die Keratinpartikel mit bipolaren Lipiden zu ummanteln, um Keratosomen zu entwickeln.

Zunächst wurden verschiedene Extraktionsmethoden an humanem Haar (alpha-Keratin) getestet. Überwiegend wurde die chemische Denaturierung zur Zersetzung des Haares genutzt. Dafür wurden Urea, Thiourea und Guanidiuniumhydrochlorid eingesetzt. Zur Unterstützung der Denaturierung fanden als Reduktionsmittel β-Mercaptoethanol, Cysteamine, L-Cystein Verwendung. Höchste Ausbeuten wurden mit einer Extraktionslösung aus 5 M Guanidiuniumhydrochlorid, 10 % Cysteamine, 25 mM Tris bei pH 8,5 erzielt. Auf Grund der Unbedenklichkeit der Substanzen Urea und L-Cystein wurde zur Isolierung des Keratins eine Extraktionslösung bestehend aus 10 M Urea, 100 mM L-Cysteine, 25 mM Tris-HCl bei pH 10,5 ausgewählt. Nach erfolgreicher Extraktion wurde die Extraktionslösung dialysiert (cutoff: 6-8 kDa, regenerierte Cellulose, SpectraPor^{®}), dabei entsteht eine kolloidale Lösung, bei hoher Proteinkonzentration und großen Dialyseschritten (5-6 I) fällt Keratin im Dialyseschlauch aus. Final wurde das Dialysat lyophilisiert, wodurch ein weißes Pulver entsteht. Dieses Pulver wurde mittels Rasterelektronenmikroskopie (REM) untersucht. Die Partikel aus Haarkeratin weisen eine variierende Größe und Form auf. Dabei wurde definiert, dass die Partikel in der finalen Formulierung eine Größe von >600 nm aufweisen sollten, um eine Penetration durch die Hornschicht der Haut zu vermeiden.

Aus regulatorischen Gründen wurde als alternative Keratinquelle Federkeratin (beta-Keratin) von verschiedenen Vogelarten verwendet. Hierzu wurden Rohfedern von Hühnern, Gänsen und Enten untersucht. Aus praktischen Erwägungen wurden insbesondere Hühnerfedern für die weiteren Untersuchungen eingesetzt.

Deshalb wurde die Extraktion von Federkeratin an den etablierten Extraktionsprozess für Haarkeratin angepasst. Dabei wurde beobachtet, dass im Vergleich zu allen anderen untersuchten Extraktionsverfahren mit der ausgewählten Extraktionslösung bestehend aus 10 M Urea, 100 mM L-Cystein, 25 mM Tris-HCl bei pH 10,5 die höchste Ausbeute zu erzielen ist. Die optionale Zugabe vom 1 M Ammoniumchlorid, verhindert die Carbamylierung am Protein, und die damit einhergehenden Veränderungen der Proteineigenschaften.

Zur Größenbestimmung wurden die Feder- und Haarkeratinpartikel vergleichend mittels Transmissionselektronenmikroskopie (TEM) im negativ Stain untersucht. Haarkeratinpartikel in kolloidaler Lösung wiesen eine Größe von ca. 40-75 nm, hingegen für die Federkeratinpartikel eine Größe von ca. 20-35 nm auf. Zur Visualisierung der Keratinpartikel für die Penetrationsuntersuchung wurden diese mit 2-Aminobenzoyl (Abz) fluoreszenz-markiert (Reaktion mit Isatosäureanhydrid unter basischen, denaturierenden Bedingungen). 5 % (w/w) fluoreszenzmarkiertes Haar- und Federkeratin wurde in Basiscreme DAC formuliert und nach Objektträgerausstrich fluoreszenzmikroskopisch untersucht. Die einzelnen Partikel aus den kolloidalen Lösungen, welche mittels TEM identifiziert wurden, bilden in Basiscreme DAC anteilig Aggregate. Für Federkeratin ist eine homogene Verteilung der zusammengelagerten Partikel zu erkennen, die Partikelgrößen liegen zwischen 20 und 35 µm. Haarkeratin hingegen lagert sich zu größeren, kristallartigen Partikeln zusammen. Die Partikelgrößen liegen hier zwischen 20 und 140 µm.

Mittels Penetrationsuntersuchungen von Haar- und Federkeratin (jeweils 5% (w/w)) an *ex vivo* Humanhaut (Franzzelle) in Basiscreme DAC konnte zudem nachgewiesen werden, dass die Keratinpartikel nicht in tiefe Hautschichten diffundieren, sondern in den oberen Anteilen des SC (Stratum disjunctum) verbleiben. Um die Interaktion der Federkeratinpartikel mit den SC-Lipiden bzw. Effekte von Lipidbeschichtungen besser einschätzen zu können, wurde bei verschiedenen pH-Werten das Zeta-Potential bestimmt (Tab. 1).

**Tab. 1: Zeta-Potential von Federkeratin und fluoreszenz-markiertem Federkeratin jeweils 10mg/ml in 10mM Kaliumchlorid bei verschiedenen pH-Werten.**

| **pH-Wert** | **Federkreatin 10 mg/mL** | **Federkreatin-Abz** |
|---|---|---|
| 10,5 | -25 mV | -24,2 mV |
| 7,6 | -1,5 mV | -23,3 mV |
| | Versuch 1: -17,8 mV | |
| 5,5 | -20,2 mV | -23,3 mV |

Die Keratinpartikel weisen eine negative Ladung auf, was sie zur Beschichtung mit positiv-geladenen Lipiden prädestiniert.

### Beispiel 2

Zur Untersuchung und Bestimmung des Einflusses von bioaktivem Keratin auf die Ordnung und somit Stabilität von SC-Lipidmodelmembranen wurden mit dem Ziel einer epikutanen Applikation mit einem aussagekräftigen Modell getestet. Um eine mögliche Interaktion auf molekularer Ebene beobachten zu können, wurden zunächst ausgewählte SC-Lipide allein und in Mischung mit CHOL und FFS in als SC- Lipidmodellmembranen in Monoschichten untersucht.

Für die Untersuchungen wurden u.a. die im nativen Stratum corneum am häufigsten vorkommenden Ceramide [AP] (*α*-hydroxy-Phytosphingosin), [NP] (nicht-hydroxyliertes Phytosphingosin) und [NH] (nicht- 6-hydroxysphingosin) allein und gemeinsam mit CHOL und der FFS Stearinsäure (SA) in einem molaren Verhältnis von 1:0,7:1 eingesetzt. In den Monoschichten wurden zudem die Einflüsse der jeweiligen Kopfgruppenstruktur der Ceramide [AS] (*α*-hydroxy-Sphingosin), [NS] (nicht-hydroxy-Sphingosin), [AdS] (*α*-hydroxy-Dihydrosphingosin) und [NdS] (nicht-hydroxy-Dihydrosphingosin) auf ihr Verhalten in der Monoschicht untersucht. Überdies wurde der Einfluss der Lipidketten auf die Stabilität der Monoschicht für die CER[AS], [AdS] und [NdS] mittels Infrarot-Reflektions-Absorptions-Spektroskopie (IRRAS) genauer verifiziert. Abschließende Röntgendiffraktionsmessungen (GIXD) lieferten nähere Aufschlüsse über die Struktur der SC- Lipidmodell-Monoschichten auf molekularer Ebene.

Nach Abschluss der umfassenden Charakterisierung der Ceramide allein und in Mischung mit CHOL und FFS als SC-Lipidmodellmembran wurde der Einfluss von Keratin auf die vorgenannten Strukturen mit denselben Methoden aufgezeigt.

### Langmuir-Trog

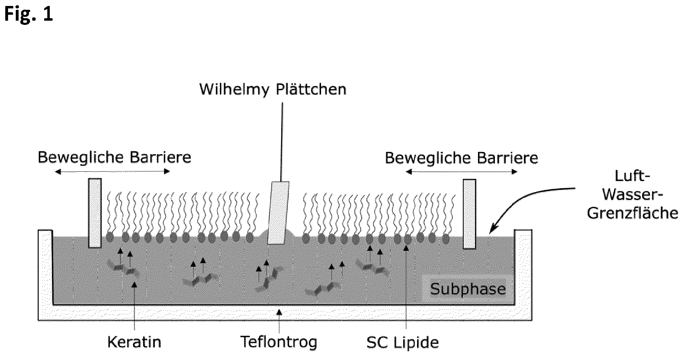

Die Untersuchungen der Interaktionen der ausgewählten Ceramide [AP], [NP], [AS], [NS], [AdS] und [NdS] allein und als SC-Lipidmodell in Mischung mit CHOL und FFS wurden zunächst unter Zuhilfenahme einer Wilhelmy Waage (oder Plättchen) auf einem Langmuir-Trog durchgeführt (Fig. 1).

Fig. 1 zeigt den schematischen Aufbau eines Langmuir-Trogs mit beweglichen Barrieren und dem Wilhelmy-Plättchen zur Bestimmung der Oberflächenaktivität.

Die Lipide wurden mit einer Hamilton Mikroliterspritze auf der wässrigen Subphase im Langmuir Trog gespreitet. Nach einer Wartezeit von 10 Minuten und der Evaporation des Lösungsmittels, wurde die Monoschicht mittels der beweglichen Barrieren mit einer Kompressionsgeschwindigkeit von 4,4 cm²/min zusammengeschoben.

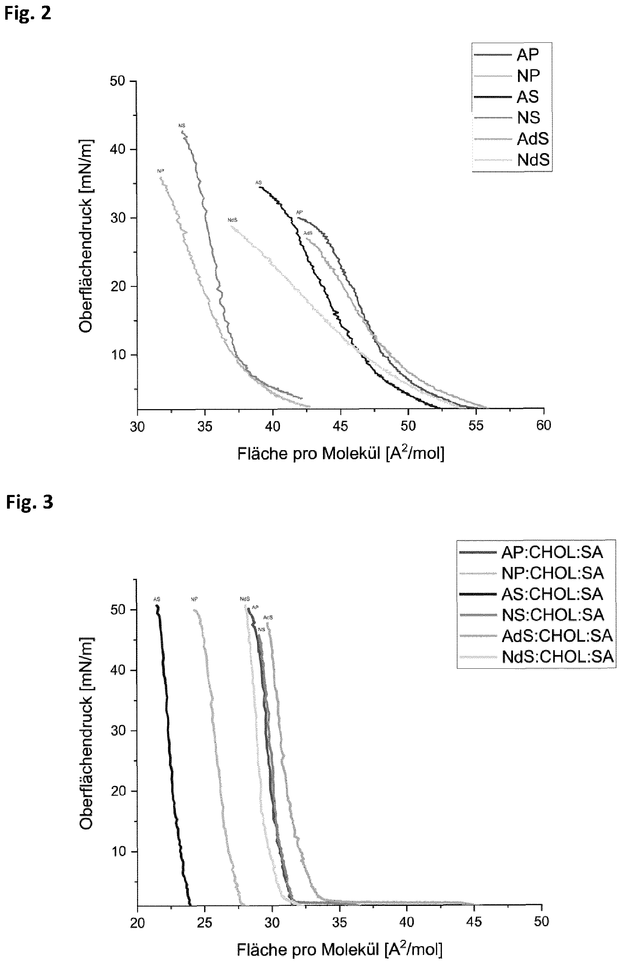

Für jedes Ceramid allein wurden drei aufeinanderfolgende Messungen durchgeführt. Die erhaltenen Isothermen (Fig. 2) wurden hinsichtlich des Lift-Off-Points, der Fläche pro Molekül bei 20 mN/m und der Kompressibilität verglichen.

Die verwendeten Ceramide unterschieden sich dabei lediglich in ihrer Kopfgruppenstruktur hinsichtlich der Anzahl und Position der Hydroxygruppen sowie dem Vorhandensein von Doppelbindungen.

Alle Isothermen beschrieben einen Übergang von einem Gas-analogen Zustand in eine festkondensierte Phase mit einer dichten Packung der Lipidmonoschicht (Fig. 2). Der Anstieg der Ceramide [AdS] und [NdS] war deutlich flacher und die erhaltene Kompressibilität deutlich kleiner als bei den anderen Ceramiden. Der weniger steile Anstieg der Monoschichten hing jedoch nicht mit einer besonders elastischen Monoschicht zusammen, sondern sprach für eine besondere Steifheit dieser Monoschichten. Im Hinblick auf den Lift-Off-Point der Isotherme zeigte sich, dass diese Ceramide eine größere Fläche pro Molekül aufwiesen als die entsprechenden Phytosphingosine [AP] und [NP], sowie Sphingosine [AS] und [NS]: [AdS] > [AP] > [AS] und [NdS] > [NP] > [NS], wie es Tabelle 1 zu entnehmen ist.

In Tabelle 1 sind die Messwerte der einzelnen Ceramide zusammengefasst.

**Tabelle 1**

| **CER** | Lift-off-point [A²] | Fläche bei 20 mN/m [A²] | Kompressibilität A |
|---|---|---|---|
| NdS | 53.30 | 40.70 | 0.05 |
| AdS | 55.70 | 44.60 | 0.07 |
| AP | 55.30 | 46.40 | 0.10 |
| AS | 49.00 | 41.50 | 0.14 |
| NP | 47.30 | 36.00 | 0.15 |
| NS | 40.60 | 36.10 | 0.22 |

Dieser Trend war ebenso bei der Fläche pro Molekül bei einem definierten Druck von 20 mN/m erkennbar. Die Kopfgruppen der Phytosphingosine nahmen durch die zusätzliche Hydroxygruppe mehr Raum auf der Grenzfläche ein, was eine mögliche Erklärung für den frühzeitigen Anstieg der Isotherme war. Zusätzlich wiesen die Ceramide mit einer α-hydroxylierten Gruppe innerhalb einer Sphingoidbase eine größere Fläche auf als die nicht-hydroxylierten Ceramide: [AP] > [NP], [AS] > [NS], [AdS] > [NdS] (s. Tabelle 1). Auch hier sorgte die zusätzliche Hydroxygruppe für eine größere Raumeinnahme der α-hydroxylierten Ceramide auf der Grenzfläche. Dass gerade die Dihydrosphingosin-basierten Ceramide [AdS] und [NdS] im Vergleich zu den anderen Sphingoidbasen die größten Flächen pro Molekül einnahmen, ließ sich nicht durch das Vorhandensein einer zusätzlichen Hydroxygruppe erklären. Hier lag es vielmehr nahe, dass sowohl die fehlende Doppelbindung als auch die fehlende Hydroxygruppe eher zu expandierten Flächen führten, da keine sterischen Interaktionen vorlagen. Die Kompressibilität der Isotherme wurde aus dem Anstieg und der Fläche bei 30 mN/m bzw. 20 mN/m bestimmt. Hier zeigten die Ceramide [AdS] und [NdS] sehr kleine Kompressibilitäten, hingegen CER [NS] die höchste. Ceramid [NdS], welches chemisch gesehen den größten Raum auf der Grenzfläche einnahm, wies die kleinste Kompressibilität auf, was bestätigte, dass seine Filme als sehr starr angesehen werden konnten. Für die Sphingosine und Phytosphingosine ließ sich anhand der Kompressibilität Folgendes ableiten: Je raumgreifender die Kopfgruppe der CERs (AP > AS > NP > NS), desto kleiner war die Kompressibilität (CER AP < AS < NP < NS) und desto starrer verhielt sich das Ceramid auf der Grenzfläche. Erkennbar war, dass die Position der zusätzlichen Hydroxygruppe an C4 bei CER[NP] der Sphingoidbase messbare Auswirkungen auf die Kompressibilität der nicht-hydroxylierten CER hatte, da für CER[NP] eine höhere Kompressibilität bestimmt wurde, wenn die OH-Gruppe in direktem Kontakt zu den anderen Gruppen an C1 und C3 stand, was auf ein Netzwerk von Wasserstoffbrücken zurückzuführen war.

Für die Mischungen der Ceramide mit CHOL und FFS zu SC-Lipidmodellmembranen zeigten die Isothermen in Fig. 3 ebenfalls einen direkten Übergang von einem Gas-analogen Zustand in eine festkondensierte Phase mit einer dichten Packung. Die Isothermen waren im Vergleich zu den Einzelkomponenten zu höheren Druckwerten komprimierbar und wiesen einen Filmkollaps auf. Die Isothermen der Einzelkomponenten (Fig. 2) zeigten dieses Verhalten nicht. Die Lift-Off-Points lagen deutlich unter denen der einzelnen Ceramide, wobei sich hier kein Zusammenhang zwischen den unterschiedlichen Kopfgruppen der Ceramide und gemessenen Flächenwerten darstellen ließ. Um eine bessere Vergleichbarkeit der Flächenwerte bei einem Druck von 20 mN/m zu schaffen, wurden über das molare Verhältnis der Flächenwerte der Einzelkomponenten (CER, Stearinsäure und Cholesterol) eine theoretische Fläche pro Molekül für die Mischungen errechnet, um herauszufinden, ob Cholesterol und Stearinsäure das Verhalten der Ceramide im Langmuir Trog beeinflussten. Die Isothermen zeigten für alle Ceramid-Mischungen, außer Ceramid [NS], eine kleinere experimentelle Fläche pro Molekül bei einem Druck von 20 mN/m. Es war demnach davon auszugehen, dass Cholesterol oder Stearinsäure das Verhalten der Kopfgruppen in der Monoschicht beeinflussten. Weiterhin lag die Kompressibilität der Isothermen über denen der Einzelkomponenten, was für weitaus weichere Monoschichten sprach. Ein Verhalten, das zu erwarten war, da hinlänglich bekannt ist, dass insbesondere CHOL für eine gewisse Fluidität von SC Lipidmembranen provoziert.

In Tabelle 2 sind die Messwerte der SC-Lipidmodellmembranen zusammengefasst.

**Tabelle 2**

| **CER** | Lift-off-point [A²] | Theoretisch berechnete Fläche bei 20 mN/m [A²] | Experimentell bestimmte Fläche bei 20 mN/m [A²] | Kompressibilität A |
|---|---|---|---|---|
| AP:CHOL:SA | 31.00 | 34.96 | 29.00 | 0.77 |
| AdS:CHOL:SA | 33.35 | 34.29 | 30.95 | 0.79 |
| NS:CHOL:SA | 33.20 | 31.14 | 31.60 | 0.81 |
| NP:CHOL:SA | 27.65 | 31.11 | 26.10 | 0.91 |
| NdS:CHOL:SA | 29.85 | 32.85 | 28.30 | 1.12 |
| AS:CHOL:SA | 23.55 | 33.14 | 22.00 | 1.36 |

### Infrarot-Reflexions-Absorptions-Spektroskopie (IRRAS)

Da der Einfluss der Kopfgruppen der Ceramide [AdS] und [NdS] aufgrund ihres rigiden Filmverhaltens in der Monoschicht uneindeutig war, wurde mittels Infrarot-Reflexions-Absorptions-Spektroskopie (IRRAS)-Messungen das Zusammenspiel ihrer Lipidketten untersucht, um so ggf. eine Erklärung für die bisherigen Ergebnisse zu erhalten. Als Vergleich zu den bisherigen Langmuir-Ergebnissen wurde das CER[AS] mitgeführt. Die Messungen erfolgten sowohl für die Monosubstanzen als auch für die SC Lipidmembranmodelle mit CHOL und FFS im Verhältnis (1:0,7:1).

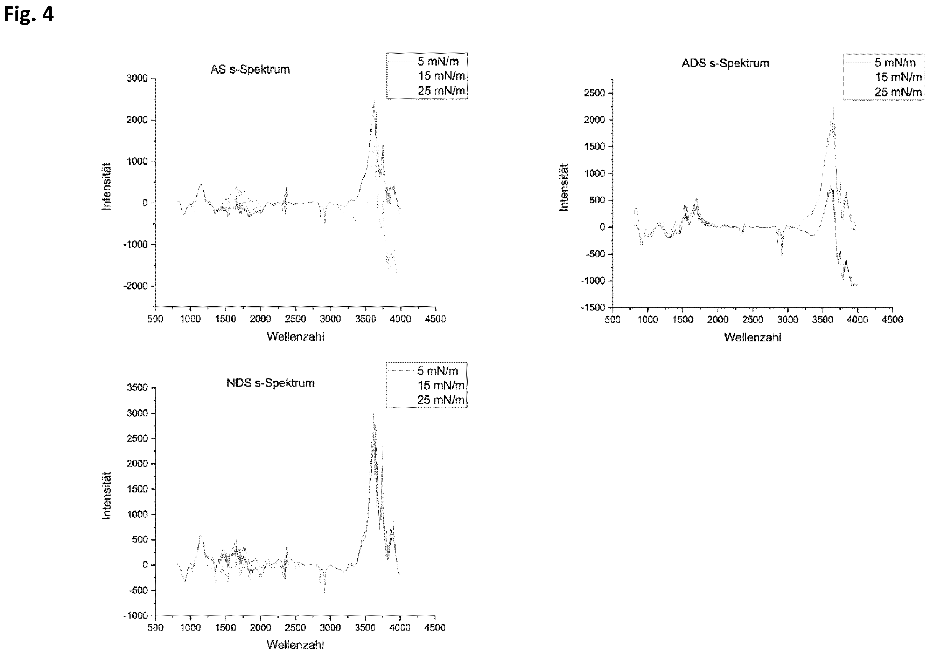

Von den Ceramiden wurde ein s-polarisiertes Spektrum aufgenommen, welches charakteristische Banden für die OH-Schwingung, symmetrische CH₂-Schwingung, die asymmetrische CH₂-Schwingung und auch CO₂ aufwies (Fig. 4). Dabei wurden die Wellenzahl und Intensität der asymmetrischen CH₂-Schwingung bei Oberflächendrücken von 5mN/m, 15 mN/m und 25 mN/m betrachtet. Bei allen Probenlösung der Einzelceramide lag die asymmetrische CH₂-Bande bei 2919,7 cm⁻¹ und demnach eine kondensierte Monoschicht vor.

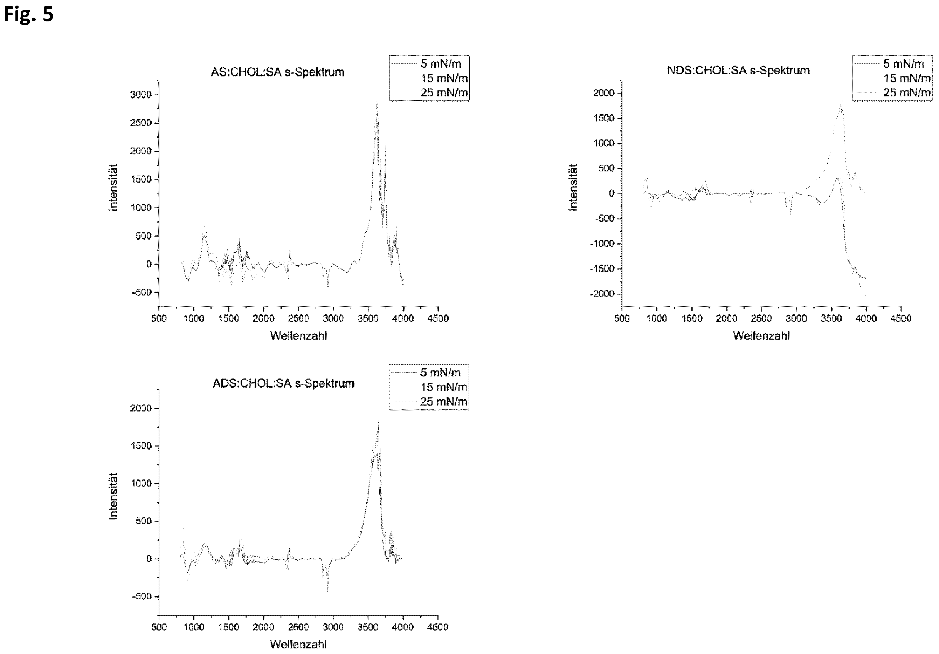

Auch die SC-Lipidmodellmembranen mit CHOL und FFS wiesen für die asymmetrische CH₂-Schwingung eine Wellenzahl von 2919,7 cm⁻¹ auf (Fig. 5). Cholesterol und Stearinsäure schienen somit keinen Einfluss auf die Kettenpackung der Lipide zu haben.

Im Anschluss an diese umfassende Charakterisierung der Ceramide und ihrer SC-Lipidmodellmischungen wurde der Einfluss von Keratin auf diese Systeme untersucht.

### Bestimmung der Oberflächenaktivität von Keratin

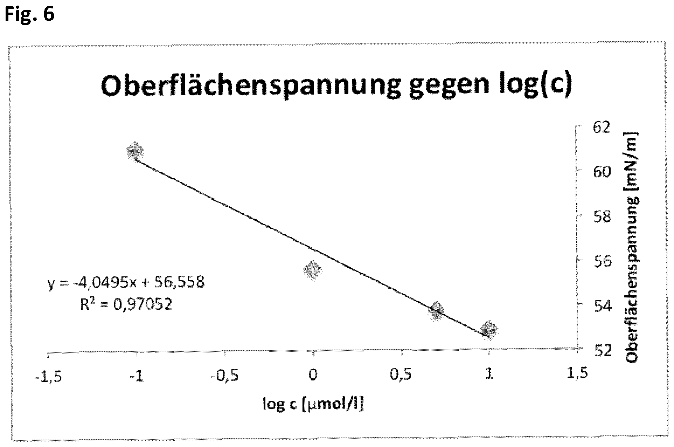

Zunächst wurde die Änderung der Oberflächenspannung von Wasser nach Zugabe des wasserlöslichen Keratins im Langmuir-Trog bestimmt. Dazu wurde kolloidale Keratinlösung in den Konzentrationen 0,1 µM, 1 µM, 5 µM und 10 µM hergestellt und in die wässrige Subphase eingespritzt. Im Anschluss daran lagerte sich das Keratin an der Grenzfläche zwischen Luft und Wasser an und setzte so die Oberflächenspannung der wässrigen Subphase herab, was mittels Wilhelmy Waage gemessen werden konnte (Fig. 6). Hier zeigte sich, dass die Oberflächenspannung abhängig von der Konzentration der Keratinlösungen war und ebenso die Zeit bis zur Einstellung der Gleichgewichtsspannung: Je höher die Konzentration des Keratins, umso schneller adsorbierten seine Moleküle an der Grenzfläche.

Bei der geringsten Konzentration von 0,1 µM stellte sich bereits bei 61 mN/m ein Gleichgewicht ein, wohingegen bei der höchsten Konzentration von 10 µM die Oberflächenspannung bis auf 52 mN/m sank. Damit wurde nachgewiesen, dass Keratin stark oberflächenaktiv war und starke amphiphile Eigenschaften besaß.

### Bestimmung von Druck-Flächen-Isothermen

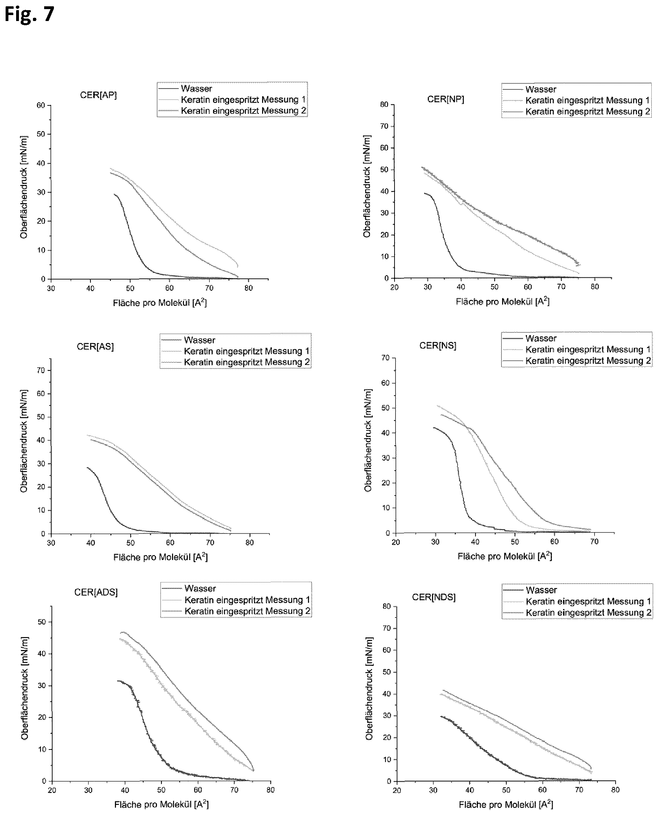

Darüber hinaus wurde der Einfluss von Keratin auf die Druck-Flächen-Isothermen ausgewählter Ceramid-Monoschichten untersucht. Zu Beginn jeder Messreihe (n=2) wurde jeweils eine Druck-Flächen-Isotherme der einzelnen CER [AS], [NS], [AP], [NP], [AdS] und [NdS] auf wässriger Phase aufgenommen und anschließend das Keratin in die wässrige Subphase injiziert (Fig. 7). Durch ein Zusammenschieben der beweglichen Barrieren des Langmuir-Trogs (Fig. 1) wurden die Ceramidfilme komprimiert, was durch die Abnahme der Fläche pro Molekül sichtbar wurde. Die Isothermen auf Keratin unterschieden sich deutlich von denen auf Wasser. Während die Isothermen der Einzelceramide auf Wasser einen charakteristischen Verlauf mit definiertem Lift-Off Point und steilem Anstieg zeigten, war dies auf keratinhaltiger Subphase nicht zu beobachten. Da der Lift-Off Point für ein Kollabieren der Monoschicht bei sprunghaftem Anstieg der Drücke steht, deutete der fehlende Lift-Off Point unter Keratineinfluss auf eine flexible Stabilisierung der Monoschicht hin, die nicht kollabierte, sondern bis zu hohen Drücken hin intakt blieb. Insgesamt sprachen auch die von Beginn an höheren Drücke bei gleicher Fläche pro Molekül für diese These, was durch eine Einlagerung des Keratins in mögliche Fehlstellen des Lipidfilms erklärbar wurde.

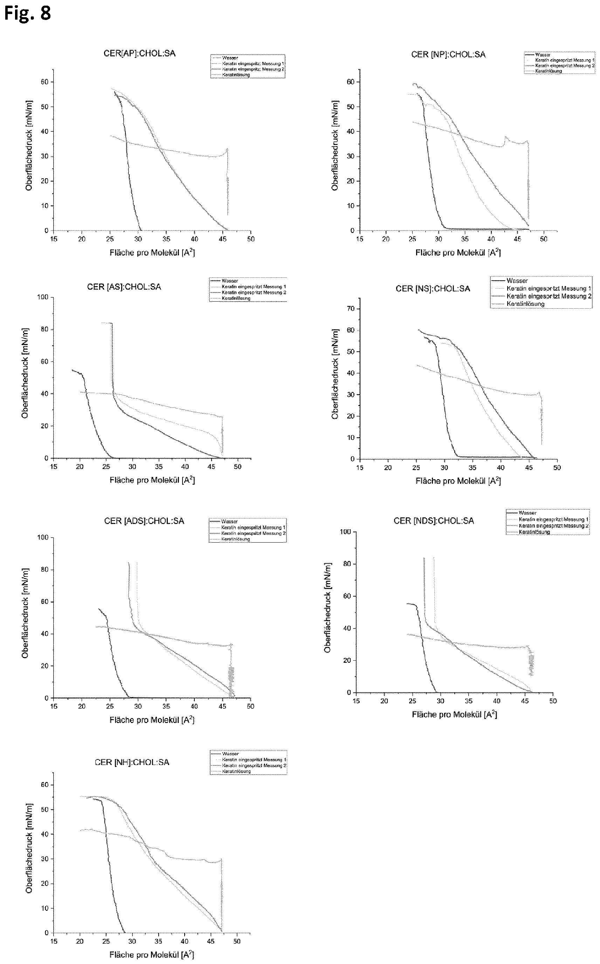

Anschließend wurde der Einfluss einer keratinhaltigen Subphase auf die SC-Lipidmodellmembranen (CER:CHOL:FFS, 1:0,7:1) im Langmuir-Trog untersucht. Auch hier unterschieden sich die Isothermen auf Keratin deutlich von denen auf Wasser (Fig. 8). Sie zeigten keinen charakteristischen Verlauf mit definiertem Lift-Off Point und steilem Anstieg. Die Flächenwerte erlaubten zudem keinen Vergleich mit den Lipidfilmen auf wässriger Phase, da die molekulare Masse des Keratins nicht genau bekannt war und somit bei den Messungen nicht berücksichtig werden konnte. Erkennbar war dennoch, dass der Oberflächendruck bereits bei größeren Flächenwerten stieg, die Isothermen ebenfalls ab einem bestimmten Oberflächendruck im Anstieg abnahmen und insbesondere bei den Mischungen der drei steifen CER [AdS], [NdS] und [AS] das Filterpapier aus der wässrigen Phase hinausgedrückt wurde, was durch den senkrechten Anstieg des Oberflächendrucks in den abgebildeten Isothermen deutlich wurde. Der Zusatz von CHOL und FFS führte hier demnach nicht zu einer Flexibilisierung der SC Lipidmodellmembran. Darüber hinaus bestätigen die vorliegenden Ergebnisse, dass Keratin keine kovalente Bindung mit den Lipiden einging. Aus den Isothermen ließ sich ableiten, dass das Keratin in mögliche Fehlstellen des Lipidfilms einlagerte, dies aber wegen fehlendem Bindungshalt bei steigender Kompression reversibel war und Keratin wieder aus den Monoschichten hinausgedrückt wurde. Mögliche andere Wechselwirkungen von nicht-kovalentem Charakter z.B. van-der-Waals-Kräfte können jedoch nicht ausgeschlossen werden. Denkbar sind zudem Wasserstoffbrückenbindungen zwischen den hydrophilen Bereichen des Keratins und der Ceramid-Kopfgruppen.

### Strukturaufklärung der SC-Lipidmodellmembranen mittels Grazing incidence X-ray Scattering (GIXD)

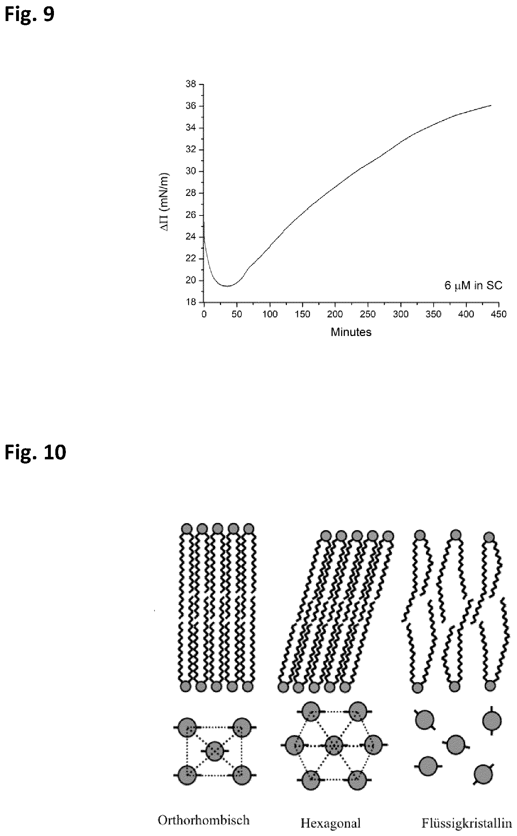

Weiterführende Untersuchungen zur Charakterisierung dieser Wechselwirkungen erfolgten mittels Röntgendiffraktometrie unter streifendem Einfall (Grazing incidence X-ray Scattering (GIXD)). In einem ersten Test ohne Röntgenstreuung wurde Keratin in einer 6 µM-Lösung als Subphase verwendet, bevor die SC-Lipidphase (CER[AP]/CHOL/FFS, 1:0,7:1) auf der Oberfläche gespreitet wurde (Fig. 9). Dabei zeigte sich eine starke Interaktion des Keratins mit der SC-Lipidmischung, da der ursprüngliche Druck von 25,5 mN/m nach Spreitung des Lipidfilms innerhalb von 30 min auf 19,5 mN/m abfiel, bevor sich über 7,5 h hinweg der Oberflächendruck des Lipidfilms auf 36,5 mN/m erhöhte, was einer Zunahme von 17 mN/m entsprach. Damit wurde deutlich, dass das Keratin den Druck auf die Lipidphase erhöhte. Es konnte also davon ausgegangen werden, dass das Keratin in der Tat Fehlstellen zwischen den Lipiden in der Monoschicht besetzte und dadurch den Oberflächendruck erhöhte.

Um diese Theorie zu bestätigen, wurden anschließend Untersuchungen per Röntgendiffraktion durchgeführt, da diese Technik eine genaue Bewertung von Fehlstellen in der Monoschicht ermöglicht. Dafür wurden SC-Lipidfilme des beschriebenen Modells CER[AP]/CHOL/FFS in einem molaren Verhältnis von (1:0,7:1) sowohl auf der wässrigen als auch auf der keratinhaltigen Subphase gespreitet, um den Einfluss des Keratins auf die laterale Ordnung der SC-Lipidmischung zu untersuchen. Dabei waren für die SC-Lipide diverse Anordnungen der lateralen Packung möglich (Fig. 10). In der Draufsicht (Fig. 10, unten) ist zu erkennen, dass in einer orthorhombischen Packung die Lipidkopfgruppen am dichtesten zueinanderstehen, wobei ihre Ketten oftmals in der lamellaren Anordnung (Fig. 10, oben) senkrecht stehen. In einer hexagonalen Packung sind größere Lücken zwischen den Kopfgruppen der Moleküle sichtbar, die z.B. durch die geneigten und parallelverschobenen Lipidketten eine größere Fläche pro Molekül einnehmen können. Dies wird insbesondere deutlich, wenn es zur Rotation der Moleküle um die eigene Achse kommt. Im Gegensatz dazu sind bei der flüssigkristallinen Anordnung kaum wiederkehrende Ordnungsparameter zu finden und die Lipide interagieren mit großem Freiheitsgrad.

Hexagonale Lipidpackungen lassen sich mittels Röntgendiffraktion durch einen Einzelpeak erkennen, wohingegen zwei Reflexe für eine orthorhombische und damit dichtere Packung der Lipide sprechen. Drei unabhängige Reflexe zeigen eine schiefwinklige Lipidanordnung an. Liegen die Reflexe auf der Q_{xy}-Achse [Å⁻¹], lässt dies auf senkrecht stehende Lipidketten schließen. Die Breite der Reflexe lässt zudem Aussagen über die Güte des Wiederholabstandes zu: Schmale, scharfe Reflexe weisen auf eine hochgeordnete und gleichmäßige Monoschicht hin, während breite Reflexe eine schlechte Korrelationslänge der Monoschicht mit zahlreichen Fehlstellen innerhalb der Einheitszelle bedeuten.

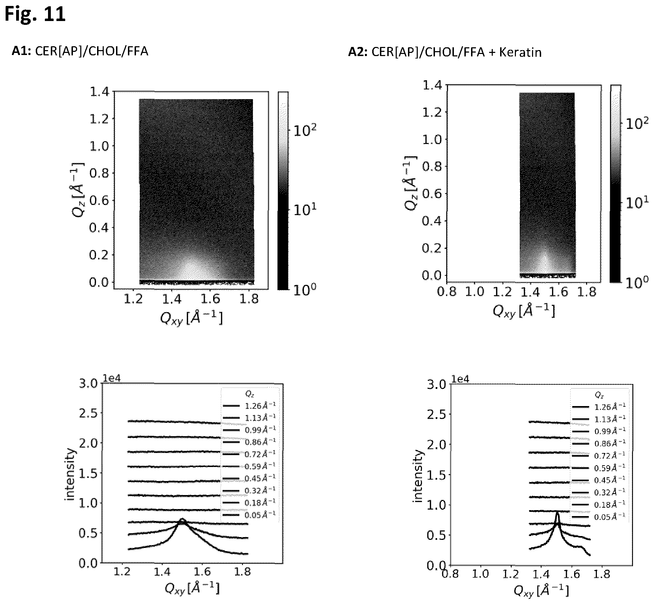

Untersucht wurden SC-Lipidmodellmonoschichten von den im nativen SC quantitativ-bedeutsamen Ceramiden CER[AP] und CER[NH] in Mischung mit CHOL und FFS (1:0,7,1). Das GIXD-Spektrum der SC-Lipidmodellmischung basierend auf CER[AP] (Fig. 11, A1, oben) zeigte einen sehr breiten und undefinierten Einzelpeak, was einer hexagonalen Packung entsprach. Auch im Intensitätsspektrum (Fig. 11, A1, unten) zeigte sich der Reflex als breiter Peak mit Schulter, was einen hohen Grad an Unordnung innerhalb der Monoschicht aufzeigt. Die GIXD-Spektren der SC-Lipidmonoschicht auf keratinhaltiger Subphase (Fig. 11, A2, oben) hingegen zeigten zwei Reflexe mit einem schärferen Reflex bei Q_{xy} = 1,5 Å⁻¹, was auf einen höheren Ordnungsgrad und weniger Fehlstellen in der Monoschicht hindeutet. Auch das Intensitätsspektrum (Fig. 11, A2, unten) verdeutlichte den intensiveren und schärferen Peak, wobei der zweite Ordnungsgrad der orthorhombischen Packung als eigenständiges Signal bei ca. Q_{xy} = 1,65 Å⁻¹ im Vergleich zum Spektrum ohne Keratin deutlich erkennbar war. Da alle Reflexe auf der Q_{xy}-Achse lagen, ist davon auszugehen, dass alle Lipidketten senkrecht stehen und Keratin in der Monoschicht keinen Einfluss auf deren Neigungswinkel hatte. Die Wechselwirkungen fanden auch hier, wie schon in den IRRAS-Messungen beobachtet, alleinig mit den Lipid-Kopfgruppen statt.

Keratin zeigte damit einen überaus komprimierenden Effekt auf die SC-Lipidmodell-Monoschicht, durch den Fehlstellen ausgeglichen und eine höhere Ordnung sowie Stabilität der Monoschicht erreicht wurde. Deutlich wurde dies durch eine Verschiebung der lateralen Ordnung unter Keratineinfluss von hexagonal zu orthorhombisch.

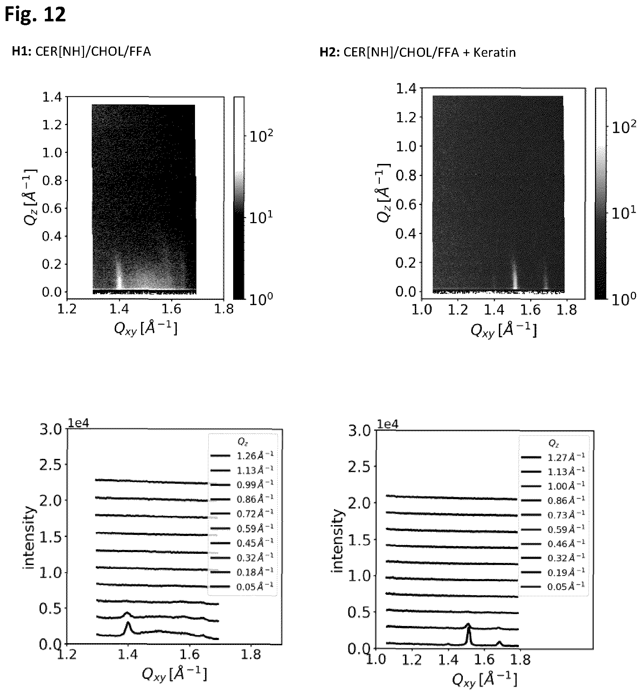

Für die CER[NH]-haltige SC-Lipidmodellmembran konnten vergleichbare Effekte beobachtet werden (Fig. 12). Das GIXD-Spektrum auf Wasser zeigte einen Hauptreflex bei 1,4 Å⁻¹ und einen schwachen zweiten Reflex bei 1,65 Å⁻¹, was einer orthorhombischen Packung zuzuordnen war. Auf keratinhaltiger Subphase verschob sich der Hauptreflex zu 1,55 Å⁻¹ auf der Q_{xy}-Achse. Er war zudem deutlich schärfer und intensiver (Fig. 12, H2, unten) als im Intensitätsspektrum auf Wasser (Fig. 12, H1, unten). Auch der zweite Reflex bei 1,65 Å¹ war deutlich erkennbar, was zusammengenommen auf eine höhere Ordnung, einheitlichere Molekülanordnungen und weniger Fehlstellen in der Monoschicht hinwies.

### Beispiel 3

### Vergleich der Wasseraufnahme und Wasserabgabe der erfindungsgemäßen topischen Formulierung im Vergleich zu Formulierungen mit hydrolysiertem Keratin

In Hygroskopie-Versuchen wurde die Wasseraufnahmefähigkeit der erfindungsgemäßen Keratinpartikel basierend auf Federkeratin, in Anlehnung an die Funktionsweise von Korneozyten, im Vergleich zu hydrolysiertem Keratin (KeraPlast^{®}, hydrolysiertes Keratin aus Schafswolle, product Pep 70, batch code: B18697, Keraplast, Neuseeland) ermittelt.

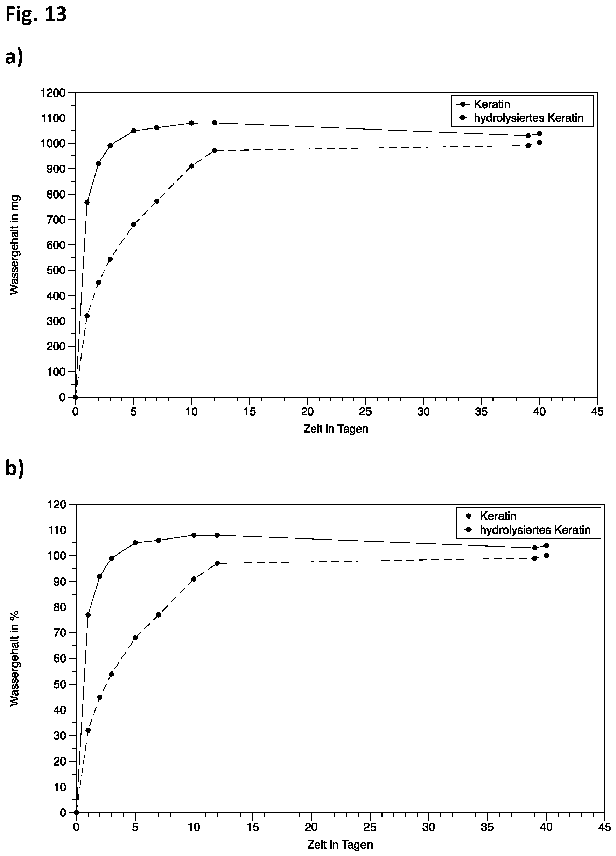

Fig. 13a und b zeigen anhand zweier Diagramme die Wasseraufnahme einer erfindungsgemäßen topischen Formulierung im Vergleich zu einer Formulierung enthaltend hydrolysiertes Keratin, wobei Fig. 13a die absolute Wasseraufnahme und Fig. 13b die prozentuale Wasseraufnahme zeigen.

Dabei zeigte sich für Federkeratin, eine Wasseraufnahme bei Raumtemperatur und 90% Luftfeuchtigkeit (Fig. 13b), mit einem Maximum ab nach 5 Tagen von ca. 100-110 %. Im Vergleich dazu wurde bei hydrolysiertem Keratin das Maximum der Wasseraufnahme von ca. 95% erst nach 12 Tagen erreicht.

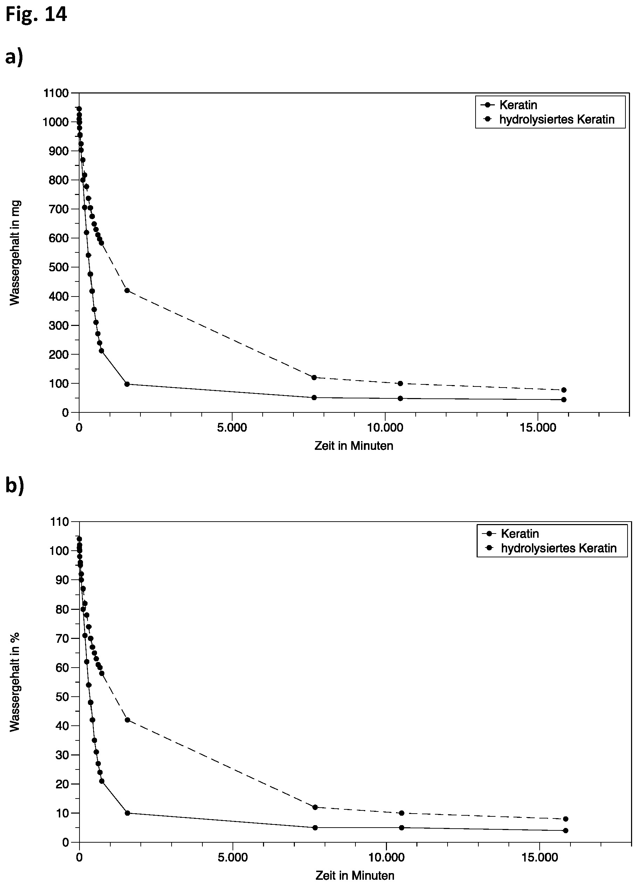

Fig. 14a und b zeigen anhand zweier Diagramme die Wasserabgabe einer erfindungsgemäßen topischen Formulierung im Vergleich zu einer Formulierung enthaltend hydrolysiertes Keratin, wobei Fig. 14a die absolute Wasserabgabe und Fig. 14b die prozentuale Wasserabgabe zeigen.

Die Wasserabgabe wurde bei Raumtemperatur und einer Luftfeuchtigkeit von 50% bestimmt und zeigte für Keratin eine rasche Wasserabgabe von ca. 80% im Vergleich zu 55% bei hydrolysiertem Keratin innerhalb der ersten 8 Stunden und anhaltend in deutlich geringerem Ausmaß für beide Präparationen bis ca. 24 Stunden (Fig. 14b).

Zusammenfassend lässt sich feststellen, dass hydrolysiertes Keratin weniger Wasser und verlangsamt aufnimmt und deutlich langsamer abgibt.

## Patentansprüche

1. Topische Formulierung enthaltend Keratinpartikel des Proteins beta-Keratin und/oder deren Agglomerate aus Federn tierischen Ursprungs zur dermatologischen Anwendung.

2. Topische Formulierung nach Anspruch 1,
**dadurch gekennzeichnet, dass** das beta-Keratin in seiner Sekundärstruktur als beta-Faltblatt vorliegt.

3. Topische Formulierung nach einem der vorhergehenden Ansprüche, **dadurch gekennzeichnet, dass** die Keratinpartikel und/oder deren Agglomerate in der topischen Formulierung eine Partikelgröße im Bereich von 10 bis 120 µm, bevorzugt von 25 bis 100 µm, gemessen mittels dynamischer Lichtstreuung, aufweisen.

4. Topische Formulierung nach einem der vorhergehenden Ansprüche, **dadurch gekennzeichnet, dass** die Keratinpartikel ein mittleres Molekulargewischt im Bereich von 5 bis 50 kDa, bevorzugt von 10 bis 30 kDa, gemessen mittels Natriumdodecylsulfat-Polyacrylamidgelelektrophorese (SDS-PAGE), aufweist.

5. Topische Formulierung nach einem der vorhergehenden Ansprüche, **dadurch gekennzeichnet, dass** die topische Formulierung Additiva enthält, bevorzugt ausgewählt aus der Gruppe bestehend aus
• Kohlenhydraten, insbesondere Saccharose, Lactose, Glucose, Fructose, Mannitol, Sorbitol und Süßstoffen wie Saccharin-Natrium, Natriumcyclamat, Aspartam, Stärke und modifizierte Stärke, Cyclodextrine und/oder Mischungen hiervon,
• Ionische Tenside, wie anionische bzw. polyanionische Tenside, insbesondere Natriumdodecylsulfat, Natriumcetylstearylsulfat, Cetylstearylalkohol (emulgierend), Natriumdioctylsulfosuccinat und/oder kationische Tenside, insbesondere Metallseifen und/oder Mischungen hiervon,
• Nichtionische Tenside, insbesondere Fettalkohole und Sterole, Sortitanfettsäureester, Polyoxyethylen-Sorbitanfettsäureester, Polyoxyethylen-Fettsäureglyceride, Macrogol-1000-glycerolmo-nofettsäureester, Polyoxyethylen-Fettsäureester, Polyoxyethylen-Fettalkoholether, Glycerolfettsäureester, Saccharosefettsäureester, Poloxamere und/oder Mischungen hiervon,
• Gelbildner, insbesondere Polyacrylate, Poloxamer, Cellulosederivate, wie Methycellulose, Methylhydroxyproylcellulose Hydroxypropylcellulose, Hydroxyethylcelluloseund/oder Ethylcellulose, Carmellose-Natrium und/oder Mischungen hiervon,
• Verdickungsmittel, insbesondere Tragant, Xantham, arabisches Gummi, Guargalactomannan, Alginate, Bentonit, und/oder Mischungen hiervon,
• Filmbildnern, insbesondere Methacrylsäure-Acrylaten, Polyvidon, Polyvinylalkohol und/oder Mischungen hiervon,
• Polymeren insbesondere Macrogole, Gelatine und/oder Mischungen hiervon,
• Konservierungsstoffe, insbesondere Methyl-4-hydroxybenzoat, Propyl-4-hydroxybenzoat, Sorbinsäure, Kaliumsorbat, Propylenglykol und/oder Mischungen hiervon,
• Antioxidativa, insbesondere Tocopherolacetat, Carotinoide, Flavonoide, Ascorbinsäure und/oder Mischungen hiervon,
• Duftstoffe
• Puffersubstanzen, insbesondere Natriumlactat, Glycolsäure und/oder Mischungen hiervon.

6. Topische Formulierung nach einem der vorhergehenden Ansprüche, **dadurch gekennzeichnet, dass** die topische Formulierung kosmetische Wirk- und Hilfsstoffe enthält, bevorzugt ausgewählt aus der Gruppe bestehend aus Aminosäuren, Peptide, Proteine, Harnstoff, Glycerol, Hyaluronsäure, Vitamine, Zuckern- und zuckerähnlichen Derivaten, Extrakten oder Wachsen aus Pflanzen oder von Tieren stammenden Teilen und Mischungen hiervon.

7. Topische Formulierung nach einem der vorhergehenden Ansprüche, **dadurch gekennzeichnet, dass** die Federn tierischen Ursprungs ausgewählt sind aus der Gruppe bestehend aus Federn von Hühnern, Gänsen, Puten, Enten, Truthähnen, Fasanen, Straußen, Nandus, Emus, Wachteln und Mischungen hiervon.

8. Topische Formulierung nach einem der vorhergehenden Ansprüche, **dadurch gekennzeichnet, dass** die dermatologische Anwendung die Stabilisierung einer Biomembran, insbesondere der Membranstruktur des Stratum corneum durch den Einbau des beta-Keratins ist, wodurch vorzugsweise eine Prophylaxe oder Therapie von Störungen der Barrierefunktion der Haut ermöglicht wird..

9. Topische Formulierung nach einem der vorhergehenden Ansprüche, **dadurch gekennzeichnet, dass** die dermatologische Anwendung eine mechanische Stabilisierung von Biomembranen, insbesondere von Ceramidmembranen des Stratum corneums, durch Erhöhung des Kollapswiderstandes der Membran durch den Einbau des beta-Keratins ist.

10. Topische Formulierung nach einem der vorhergehenden Ansprüche, **dadurch gekennzeichnet, dass** die dermatologische Anwendung eine Stabilisierung der äußeren Schicht des Tränenfilms durch den Einbau des beta-Keratins ist.

11. Topische Formulierung nach einem der vorhergehenden Ansprüche, **dadurch gekennzeichnet, dass** die dermatologische Anwendung an Lebewesen, insbesondere an Menschen und/oder Tieren erfolgt, insbesondere für die Haut, Schleimhaut oder Hautanhangsgebilde, bevorzugt Nägeln und Haaren.

12. Topische Formulierung nach einem der vorhergehenden Ansprüche in Form von
• gasförmige Grundlagen, insbesondere Aerosole und/oder Gasaphronen,
• flüssige Grundlagen, insbesondere Lösungen, Emulsionen, Suspensionen, Schäume und/oder Kolloiden,
• halbfeste Grundlagen, insbesondere Salben, Cremes, Gele, Pasten, Kolloiden und/oder Suppositorien oder
• feste Grundlagen, insbesondere Pulver, Puder, Tabletten, Granulate, Pellets, Kapseln, Pflaster, Wundauflagen und Verbandsmittel, Textilfasern und/oder Inserts.

13. Verwendung von Keratinpartikeln des Proteins beta-Keratin und/oder deren Agglomerate aus Federn tierischen Ursprungs zur Herstellung von topischen Formulierungen für die dermatologische Anwendung.
